Europäisches Patentamt
European Patent Office
Office européen des brevets

(19)

(11) Publication number: **0 423 956 A1**

# (12) EUROPEAN PATENT APPLICATION

(21) Application number: **90310531.0**

(22) Date of filing: **26.09.90**

(51) Int. Cl.5: **C30B 29/54**, C30B 23/02, C07C 255/31, C23C 14/12

The title of the invention has been amended (Guidelines for Examination in the EPO, A-III, 7.3).

(30) Priority: **26.09.89 JP 249906/89**

(43) Date of publication of application: **24.04.91 Bulletin 91/17**

(84) Designated Contracting States: **DE FR GB**

(71) Applicant: **SEMICONDUCTOR ENERGY LABORATORY CO., LTD.**
**398 Hase**
**Atsugi-shi Kanagawa-ken, 243(JP)**

(72) Inventor: **Miyanaga, Akiharu**
**Coop Denen 2-6, 1-5-5, Nagao, Tama-ku**
**Kawasaki-shi, Kanagawa-ken, 214(JP)**
Inventor: **Hongo, Masashi**
**Flat Sel 105, 304-1, Hase**
**Atsugi-shi, Kanagawa-ken, 243(JP)**

(74) Representative: **Milhench, Howard Leslie et al**
**R.G.C. Jenkins & Co. 26 Caxton Street**
**London SW1H 0RJ(GB)**

## (54) Method for producing charge transfer complex crystals from the vapor phase.

(57) A charge transfer complex of donor and acceptor molecules is formed by a vapor phase reaction, and after formation the complex is subjected to thermal annealing in order to optimize the proportions of donor and acceptor molecules. By virtue of this method, the formation speed is significantly increased.

EP 0 423 956 A1

# IMPROVEMENTS RELATING TO CHARGE TRANSFER COMPLEXES

## FIELD OF THE INVENTION

The present invention concerns improvements relating to charge transfer complexes, and more particularly concerns an improved method of forming organic charge transfer complexes.

## BACKGROUND OF THE INVENTION

A considerable amount of research has been carried out into the formation of charge transfer complexes of low molecular weight compounds and many such complexes have been obtained. In such complexes electric charge is exchanged between constituent donor molecules (represented hereinafter simply by D) supplying electrons and constituent acceptor molecules (represented hereinafter simply by A) receiving electrons. Some complexes can be formed which have high electrical conductivities depending upon the amount of charge transfer that is effected.

Among known useful acceptor molecules are TCNQ (tetracyanoquinodimethane) and derivatives thereof, for example:

TCNQ: tetracyanoquinodimethane:

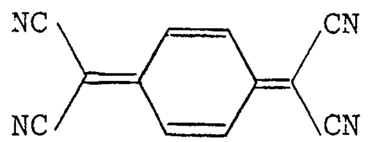

$(Me)_2$TCNQ:

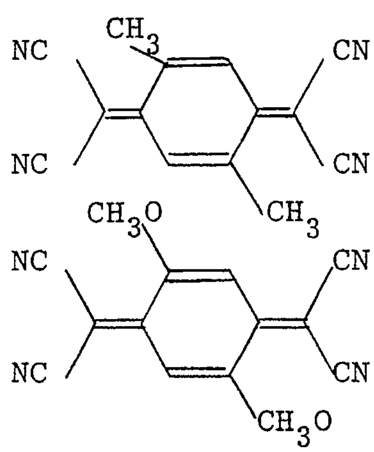

$(MEO)_2$TCNQ:

These molecules have been known to possess large electron affinities and form charge transfer complexes with many donor molecules. Other acceptor molecules are as follows:

TNAP: tetracyanonaphtoquinodimethane:

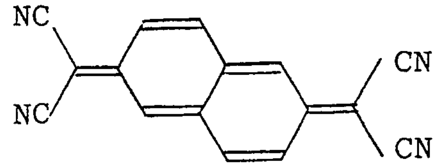

TCNDQ:

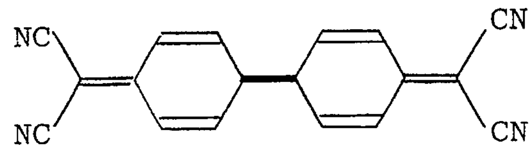

Acceptor molecules of these kinds, and including TNAP, will hereinafter be referred to as TCNQ-based molecules.

Among known useful donor molecules are, for example:

TTF: tetrathiafulvalene:

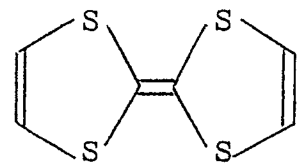

TMTTF: tetramethyltetrathiafulvalene:

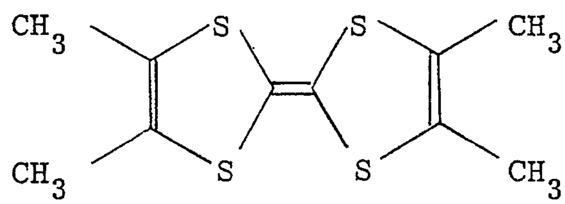

TSF: tetraselenafulvalene:

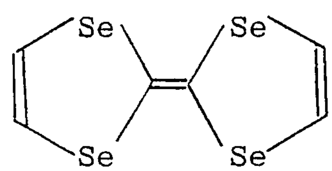

TMTSF: tetramethyltetraseleafulvalene:

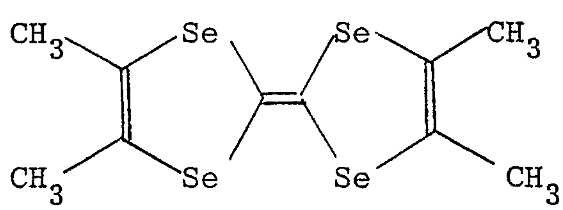

Donor molecules of this kind are called simply TTF-based molecules.

In the molecular structures of TTF-based molecules and TCNQ-based molecules, central atoms are arranged in a plane constituting the backbone of the molecule (called hereinafter the molecular plane). A number of $\pi$ electrons existing in the molecular plane play determinant functions for the characteristics of the substance.

Electron transfer complexes can be obtained by combining suitable donor and acceptor molecules, for example, by combining the above described TTF-based and TCNQ-based molecules such as $(TTF)_x$ - $(TCNQ)_y$, $(TMTTF)_x$ $(TCNQ)_y$, $(TSF)_x$ $(TCNQ)_y$ and $(TMTSF)_x$ $(TCNQ)_y$. Several tens or thousands of S/cm of conductivity may be achieved by selecting suitable preparation methods.

In order to form charge transfer complexes having such a high electrical conductivity as to seem almost metallic, there are two requirements as follows. First, the ratio between x and y in $D_xA_y$ has to be optimized

by, e.g., eliminating impurities to avoid disturbance of the crystalline structure due to the existence of impurities. In the case of complexes of the type (TTF-based molecule)$_x$ (TCNQ-based molecule)$_y$, the ratio has to be adjusted so that x:y = 1:1 in order to obtain the desired quasi-metallic complexes, while complexes become semiconducting at x:y = 1.3:2 or 1.66:2. Second, donor molecules and acceptor molecules have to be arranged into a "separated laminate" in which donor and acceptor molecules are laminated in different planes which are not parallel with each other as illustrated in Fig.1(A) of the accompanying drawings where the solid lines represent the molecular planes of respective molecules. When a complex is prepared in this laminar structure, there are formed separate molecular columns consisting of donor molecules and acceptor molecules respectively. The molecular columns form respective current paths in the complex so that its conductivity is increased. For this reason, it is very important for obtaining complexes having high conductivities to construct their crystalline structures selectively as separated laminates.

Attempts have been made to form charge transfer complexes generally by utilizing liquid state reactions of solvents, such as by diffusion methods or electric field methods. The preparation time required to form complexes by such conventional methods is however very long; for example it may be several months or longer in the case of the diffusion method. When prepared over such a long time, the donor molecules and acceptor molecules tend to be arranged into an overlapping laminar structure in which the donor and acceptor molecules are arranged in parallel and partially and alternately overlapped as illustrated in Fig.1(B) of the accompanying drawings, rather than being separate laminates. This is because the overlapping laminate constitutes the stable state of the complex, while the separated laminate constitutes only a metastable state.

For example, it has been reported that when (TMTSF)$_x$(TCNQ)$_y$ was formed by liquid phase diffusion at x:y = 1:1, only reddish crystals corresponding to the overlapping laminate were formed when the formation time was long, while black crystals corresponding to the separated laminate were obtained when the formation time was relatively short. From these considerations, it seems preferable to decrease the time spent for the formation of complexes in order to achieve high conductivities.

There are some exceptions in that complexes having relatively high conductivities can in fact be formed by conventional liquid phase methods. For example, (TMTTF)$_x$(TCNQ)$_y$ can be formed by a liquid phase method at x:y = 1:1 so as to result in a quasi-metallic phase with a black crystalline appearance. This method is, however, hardly applicable for commercialization (mass-production) since the preparation time is too long, for example, several months or longer as in the case of the liquid diffusion methods described above. When prepared within a shorter time, the product assumes the form of microcrystals which can be utilized for only very few applications. In addition, the control of complex crystal formation by such methods is too difficult to enable the appropriate separated laminates required to realize sufficiently high conductivities for practical applications to be reproduced.

## BRIEF SUMMARY OF THE INVENTION

In accordance with the present invention, the basic separated laminar structure of a charge transfer complex is constructed in a vapor phase reaction, and this is followed by an annealing process which eliminates unnecessary substances from the basic structure and makes the laminate perfect. By virtue of this method improved charge transfer complexes can be manufactured with ease and, more particularly, large amounts of charge transfer complex can be formed at a high speed suitable for mass production.

As is explained in detail hereinafter with reference to a specific embodiment of the invention, in a first step the basic separated laminate structure comprising donor molecules D and acceptor molecules A of a charge transfer complex $D_xA_y$ is constructed by a direct vapor phase reaction involving sublimation or melting and evaporation. The donor source material and the acceptor source material may be processed concurrently or sequentially, or a precursor material may be prepared by conducting an advance reaction between the donor and acceptor materials and using the resulting precursor material as a single source in the vapor phase reaction.

The complex crystals formed by the vapor phase reaction are annealed at an appropriate temperature at which thermal decomposition will not take place, for example 100-150 °C or lower, for several hours in order to eliminate contaminants and optimize x and y in $D_xA_y$ for the purpose of making the crystallization perfect. The values of x and y in $D_xA_y$ approach target values (1:1) as the annealing time increases. Depending upon the particular complex being processed, however, the molecular structure of the complex may be converted from a separated laminate to an overlapping laminate structure if the annealing at high temperature is continued for too long. Usually, the annealing time is up to 10 hours, though usable crystals

may be formed after only 1 hour of annealing in some cases. The annealing is preferably carried out in an apparatus which is designed not to allow undesired substances that have been removed from the crystals to get mixed therewith again, and is preferably carried out at a reduced pressure (approx. 10 Torr or lower) and/or in an atmosphere of an inactive gas such as Ar and $N_2$ which may be flushed through the annealing chamber.

The crystals obtained by performance of the above method are small black single crystals or polycrystalline films. The values of x and y in $D_xA_y$ are optimized so that high conductivities are exhibited.

Both the donor and the acceptor source materials are preferably purified in advance of the actual preparation of the charge transfer complex. This treatment is desirable in order to obtain complex crystals having high purity. The high purity obtained by purification of an organic substance is lowered in general only be continuing exposure to air and therefore the purification should preferably be carried out just before the vapor phase reaction. Some donor or acceptor materials decompose at a temperature lower than their sublimation or melting and evaporation temperatures and the present invention can not be applied to such donor or acceptor materials; it can readily be ascertained in the purification process whether or not the selected donor or acceptor materials can be used.

Further features of the present invention are set forth with particularity in the appended claims and, together with the features abovementioned, will best be appreciated from ccnsideration of the following detailed description given with reference to the accompanying drawings.

## BRIEF DESCRIPTION OF THE DRAWINGS

Fig. 1(A) is a schematic diagram showing a separated laminate molecular arrangement;
Fig. 1(B) is a schematic diagram showing an overlapping laminate molecular arrangement;
Fig. 2 is a cross-sectional view showing a vapor phase reaction apparatus suitable for carrying out methods in accordance with a first embodiment of the present invention;
Fig. 3 is a cross-sectional view showing a vapor phase reaction apparatus suitable for carrying out methods in accordance with a second and a third embodiment of the present invention; and
Fig. 4 is a cross-sectional view showing a thermal annealing apparatus suitable for carrying out methods in accordance with a fourth embodiment of the present invention.

## DETAILED DESCRIPTION OF THE EMBODIMENTS

Referring now to Fig. 2 of the drawings which illustrates an apparatus suitable for carrying out vapor phase reactions, a method of preparing charge transfer complexes in accordance with a first embodiment of the invention will now be described. The apparatus comprises a reaction tube 1, retainers 5 and 6 provided inside the tube 1 for the donor and acceptor materials. Teflon ™ substrates 8 attached to the inside of the tube 1, a ceramic cylinder 2 supporting the reaction tube 1, an electric heater 3 surrounding the cylinder 2 and a chromel-alumer thermocouple 7. The reaction tube 1 and the retainers 5 and 6 are made form Pyrex ™ glass. The locations of the substrates 8 and of the retainers 5 and 6 and the desired temperature to be detected by the thermocouple 7 are determined in accordance with the sublimation temperatures of the donor and acceptor source materials and with reference to the temperature gradient in the vertical direction. Examples of sublimation temperatures of donors and acceptors are as follows:
TTF: 111 °C (5 mmHg), TMTTF: 157 °C (5 mmHg),
TMTSF: 164 °C (3 mmHg), TCNQ: 180 °C (11 mmHg)

After putting donor and acceptor source materials respectively in the retainers 5 and 6 and evacuating the inside of the tube 1 to an appropriate pressure, the tube 1 was hermetically closed and heated by means of the heater 3. The heating was continued for approximately an hour. As a result, $D_xA_y$ complex crystals 4 of 100~200 $\mu$m width and 1 mm height were grown on the Teflon substrates 8. The total weight of the crystals was approximately 100 mg.

The $D_xA_y$ complex crystals thus formed were electrically conductive but exhibited the characteristics of a semiconductor with a ratio of $x:y \neq 1:1$. These crystals were thermally annealed at 100~150 °C for 2 hours in a muffle furnace. As a result, the desired charge transfer complexes were obtained and examples of the electric conductivities that were obtained are shown below.
TTF•TCNQ: 500 S/cm, TMTTF•TCNQ: 60 S/cm
TSF•TCNQ: 5200 S/cm, TMTSF•TCNQ: 100 S/cm

Although in the above example the tube 1 was hermetically sealed off, the donor and acceptor materials can be supplied into an open tube in fluid form from bubbling devices in which liquids containing donor and

acceptor source materials are bubbled through by an inert gas. The fluid donor and acceptor source materials are mixed in the open tube so that complex crystals are grown on the substrate. Although in the structure shown in Fig. 2 the substrate on which crystals grow is located to be heated, the location may be a cooling location in some particular cases.

Referring to Fig. 3, a second embodiment of an apparatus for carrying out the present invention will now be described. The figure illustrates a vapor reaction apparatus comprising a vacuum chamber 13, a rotary vacuum pump 12 connected to the chamber 13, a Teflon substrate 11 provided with a heater 14 in the chamber 13 and retainers 9 and 10. The retainers 9 and 10 are also provided with built-in heaters and both retainers 9 and 10 and the substrate 11 are provided with chromel-alumer thermocouples in order to enable their temperatures to be controlled. TTF or TMTT as a donor source and TCNQ as an acceptor source were put in the retainers 9 and 10. The inside of the chamber 13 was evacuated by means of the rotary pump 12 to a pressure of no higher than 1 Torr. Crystals of $D_xA_y$ were grown on the substrate 11 by heating the retainers 9 and 10. The ratio of x:y was controlled by regulating the opening of shutters 15 provided at the outlets of the retainers 9 and 10 as well as by adjusting the temperatures of the retainers 9 and 10. The temperatures of the retainers 9 and 10 were determined with reference to the sublimation temperatures of the donor and acceptor materials. The opening of each shutter 15 was controlled by an automatic regulating device which determined how many times the shutter was opened per minute, the shutter being opened for about one second at a time. During the formation of crystals, the temperature of the substrate 11 was maintained at a certain temperature selected to be between room temperature and 80° C, and this temperature was changed as different samples were investigated.

By trial and experiment it has been determined that the temperature of the substrate 11 should be between 25° C and 40° C. At a lower temperature, the crystallization speed decreased and the size of the crystals was reduced. The appropriate opening time (total time) of the retainer 9 holding the acceptor material was equal to or about 10% shorter than that of the other retainer 10 holding the donor material. With appropriate conditions thus determined, a large number of black complex crystals were grown to 1 mm or near 1 mm height in a 60-120 minute process. The ratio of x:y was near 1:1. The electrical conductivity was relatively high, while some semiconductive nature was apparent.

The crystals thus formed were subjected to thermal annealing in an incubator through which nitrogen was passed. After the annealing had continued for 5 hours at 300° C, the ratio of x:y was improved to be just 1:1. The conductivities were measured to be 600 S/cm for TTF•TCNQ and 70 S/cm for TMTTF•TCNQ. The structure of these crystals are considered to be a completely separated laminate.

A method in accordance with third embodiment of the present invention was also carried out by use of the apparatus illustrated in Fig. 3. The heater 14, however, was replaced by another heater capable of heating the substrate to about 150° C. n this embodiment, the formation of complex crystals was carried out in the same manner as in the second embodiment except that the annealing process was carried out in situ in the vacuum chamber 13 at 100° C or lower temperatures for three hours and that TMTSF was used instead of TMTTF. The conductivities were measured to be 100 S/cm for TTF•TCNQ and 200 S/cm for TMTTF•TCNQ. In regard to TMTTF•TCNQ, when the annealing temperature was lower than 100° C the conductivity was slightly higher than that achieved at 100° C. In accordance with this embodiment, the effect on the separated laminate structure of its exposure to impurities during transportation of the complex crystals from the crystal formation stage to the thermal annealing stage could be avoided.

Next, a fourth embodiment will be described. The formation of complex crystals of this embodiment was carried out in the same manner as in accordance with the second embodiment except that TMTSF was used instead of TMTTF. The thermal annealing was carried out in an annealing apparatus as illustrated in Fig 4. This annealing apparatus comprises a vacuum chamber 21, a rotary vacuum pump 20 and a diffusion pump 19 connected to the vacuum chamber 21, a substrate provided with a heater 22 in the chamber 21 and a pair of jet nozzles 18 and 17 inserted into the inside of the chamber toward the substrate 16. After transporting the complex crystals together with the substrate 8 from the apparatus shown in Fig. 3 to the upper surface of the substrate 16, the chamber was evacuated to 0.1 Torr or a lower pressure followed by heating the substrate 16 to 100° C. The annealing was continued for an hour during the annealing process, a jet of inactive gas was caused to flush away impurities driven out of the crystals by the annealing effect. In order to avoid any undesirable direct influence of the jet upon the crystals, the distances between the crystals and the end of each of the nozzles 17 and 18 were selected to be no shorter than 1 cm. In accordance with this embodiment, it was ensured that no undesirable influence from impurities driven out from the complex crystals during thermal annealing could be exerted upon the separated laminate structure. As a result, a large number of black complex crystals were grown to approximately 1 mm with the ratio of x:y = 1. The electrical conductivities were measured to be 400 S/cm for TTF•TCNQ and 150 S/cm for TMTTF•TCNQ.

The foregoing description of preferred embodiments has been presented for purposes of illustration and description. It is not intended to be exhaustive or to limit the invention to the precise form described, and obviously many modifications and variations are possible without departure from the scope of the invention. For example, whereas in a described embodiment the amounts of donor and acceptor material were controlled by provision of shutters, the sizes of the outlets or each retainer may be predetermined in order to obtain the desired 1:1 ratio.

## Claims

1. A method of forming a charge transfer complex of donor and acceptor molecules, said method comprising:
forming crystals of the complex by means of a vapor phase reaction; and
thermally annealing said crystals in order to optimize the proportions of said donor and acceptor molecules in said complex.
2. The method of claim 1 wherein the formation of said crystals is effected by vaporizing a solid state source of said donor and acceptor molecules at an elevated temperature and growing crystals of said complex from then resulting vapor.
3. The method of claim 2 wherein said solid state source is disposed in a vacuum chamber and heated to produce vapor and crystals of said complex are grown on a substrate.
4. The method of claim 2 or 3 wherein donor and acceptor source materials are reacted together to produce a precursor donor/acceptor material which is then processed to form a vapor from which crystals are grown.
5. A method of claim 2 or 3 wherein said source comprises a donor source material and an acceptor source material contained in separate retainers.
6. A method of claim 5 wherein the sizes of the outlets of said retainers are adjusted to achieve the appropriate proportions of donor and acceptor molecules in said complex.
7. The method of claim 5 wherein the amount of donor and acceptor material that is vaporized is controlled by means of shutters provided at the outlets of said retainers to achieve the appropriate proportions of donor and acceptor molecules in said complex.
8. The method of any preceding claim wherein said donor is TTF, TMTTF, TSF or TMTSF.
9. The method of any preceding claim wherein said acceptor is TNAP, TCNDQ or TCNQ or its derivative.
10. The method of any preceding claim wherein said complex is $(TTF)_x(TCNQ)_y$, $(TMTTF)_x(TCNQ)_y$, $(TSF)_x$-$(TCNQ)_y$ or $(TMTSF)_x(TCNQ)_y$.
11. The method of claim 1 wherein said forming step comprises bubbling liquids containing said donor and said acceptor with an inactive gas and heating said inactive gas carrying said donor and acceptor to carry out said vapor phase reaction.
12. The method of any preceding claim wherein said crystals are formed on a Teflon substrate.
13. The method of any preceding claim wherein the formation of the crystals is carried out in a vacuum chamber and is followed by the annealing step which is performed in the same vacuum chamber by heating a substrate on which the crystals are formed.
14. The method of any preceding claim wherein the surfaces of said crystals are flushed by an inactive gas during said annealing.
15. The method of any preceding claim wherein annealing is carried out at a temperature of from 100 to 150 °C.

FIG. 1(A)

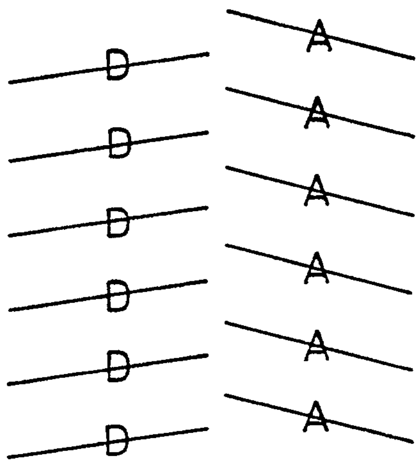


FIG. 2

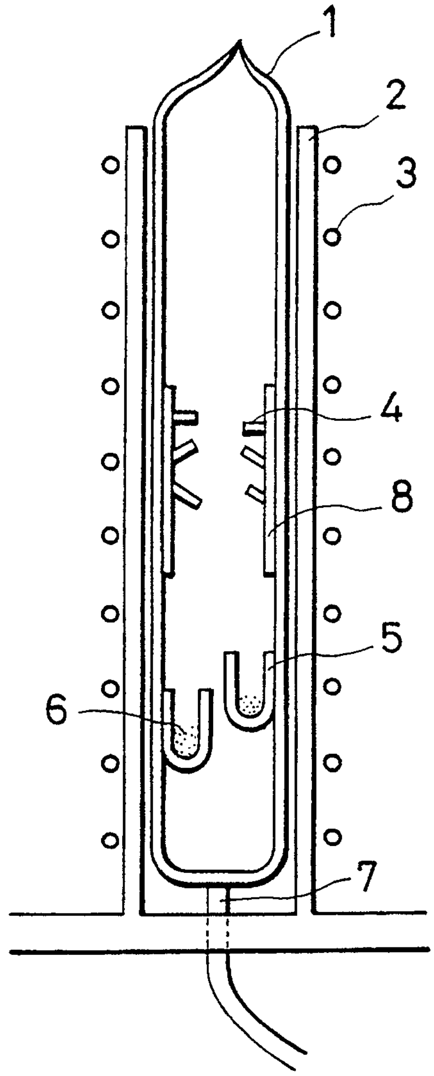


FIG. 1(B)

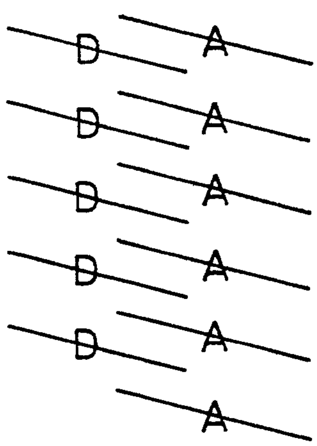

FIG. 3

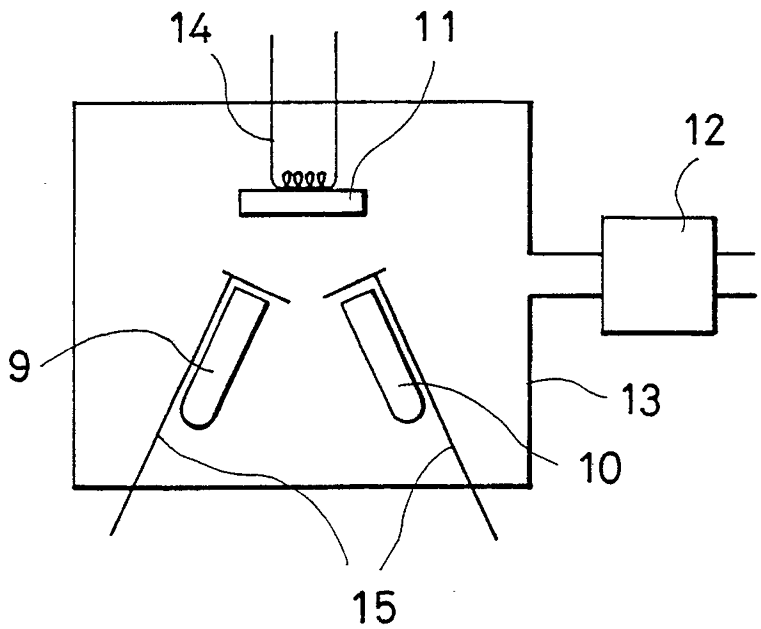


FIG. 4

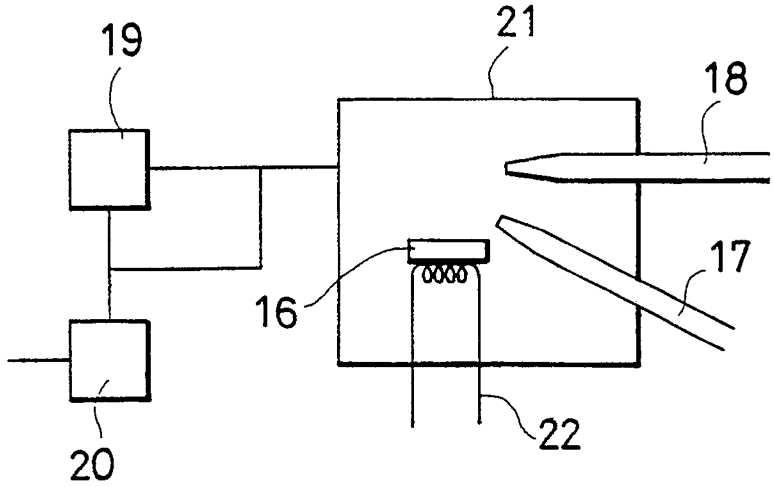

European Patent Office

# EUROPEAN SEARCH REPORT

Application Number

**EP 90 31 0531**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF CRYSTAL GROWTH, vol. 26, 1974, pages 187-196, Amsterdam, NL; Y. MURATA et al.: "Topotaxial formation of an anthracene-TCNQ (1:1) complex in the solid phase" * Pages 189-190 * | 1,9,13 | C 30 B 29/54<br>C 30 B 23/02<br>C 07 C 255/31<br>C 23 C 14/12 |
| A | PATENT ABSTRACTS OF JAPAN, vol. 12, no. 349 (C-529)[3196], 20th September 1988; & JP-A-63 105 965 (CANON INC.) 11-05-1988 * Whole abstract * | 1-3,5, 7-10 | |
| A | IBM TECHNICAL DISCLOSURE BULLETIN, vol. 19, no. 4, September 1976, pages 1398-1399, New York, US; J. DURAN: "Vapor growth of homogenous binary/tertiary organic crystals" | | |
| A | IBM TECHNICAL DISCLOSURE BULLETIN, vol. 17, no. 1, June 1974, pages 303-305, New York, US; R. BRACCINI et al.: "Sublimation growth of organic charge transfer salt crystal films and bulk crystals" | | **TECHNICAL FIELDS SEARCHED (Int. Cl.5)** |
| A | IBM J. OF RESEARCH AND DEVELOPMENT, vol. 22, no. 3, May 1978, pages 315-319, New York, US; E.E. SIMONYI et al.: "Oriented epitaxial films of (NMP)(TCNQ)" | | C 07 C<br>C 23 C<br>C 30 B |

The present search report has been drawn up for all claims

| Place of search | Date of completion of search | Examiner |
|---|---|---|
| The Hague | 22 January 91 | COOK S.D. |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document